# EUROPÄISCHE PATENTSCHRIFT

(11) **EP 1 188 055 B1**
(45) Veröffentlichungstag und Bekanntmachung des Hinweises auf die Patenterteilung: **14.04.2004**
(21) Anmeldenummer: 00929493.5
(22) Anmeldetag: 04.05.2000
(51) Int. Cl.: G01N 33/50, C12P 13/24

(54) **LABORMÄSSIGE UNTERSUCHUNG EINER KÖRPERFLÜSSIGKEITS- ODER GEWEBEPROBE**
LABORATORY EXAMINATION OF A BODY FLUID OR TISSUE SAMPLE
EXAMEN EN LABORATOIRE D'UN ECHANTILLON DE LIQUIDE ORGANIQUE OU DE TISSU EFFECTUE

(30) Priorität: 04.05.1999 DE 19920434
(43) Veröffentlichungstag der Anmeldung: 20.03.2002
(73) Patentinhaber: Hoerrmann, Wilhelm, Dr., 82393 Iffeldorf (DE)
(72) Erfinder: Hoerrmann, Wilhelm, Dr., 82393 Iffeldorf (DE)
(74) Vertreter: Grättinger, Günter
(86) Internationale Anmeldenummer: PCT/EP2000/004003
(87) Internationale Veröffentlichungsnummer: WO 2000/067022

(56) Entgegenhaltungen:
- WO-A-97/33578
- DATABASE BIOSIS [Online] BIOSCIENCES INFORMATION SERVICE, PHILADELPHIA, PA, US; 1996 MORI HIDEO ET AL: "Detection of novel proline 3-hydroxylase activities in Streptomyces and Bacillus spp. by regio- and stereospecific hydroxylation of L-proline." Database accession no. PREV199699037561 XP002154373 & APPLIED AND ENVIRONMENTAL MICROBIOLOGY, Bd. 62, Nr. 6, 1996, Seiten 1903-1907, ISSN: 0099-2240
- DATABASE BIOSIS [Online] BIOSCIENCES INFORMATION SERVICE, PHILADELPHIA, PA, US; 1984 BIENKOWSKI R S: "A CRITERION TO DETERMINE WHETHER CIS-4 HYDROXY PROLINE IS PRODUCED IN ANIMAL TISSUES" Database accession no. PREV198478044739 XP002154374 & ARCHIVES OF BIOCHEMISTRY AND BIOPHYSICS, Bd. 229, Nr. 2, 1984, Seiten 455-458, ISSN: 0003-9861

## Beschreibung

Die Medizin kennt den Begriff der Krankheitsprädisposition, was bedeutet, daß bei manchen Menschen die Wahrscheinlichkeit, eine bestimmte Krankheit zu erwerben, größer ist als bei anderen. Kommt es aufgrund einer solchen Prädisposition, die vielfach genetisch bedingt ist, zu einer Erkrankung, ohne daß diese bereits klinische Symptome auslöst, so ist die entsprechende Erkrankung häufig entweder überhaupt nicht, oder nur mit sehr aufwendigen und belastenden Methoden nachweisbar.

Damit die Durchführung solcher aufwendiger und belastender Methoden auf Fälle beschränkt werden kann, in denen eine hohe Wahrscheinlichkeit für das Vorliegen einer Krankheitsprädisposition besteht, wäre es wünschenswert, ein Indikator für eine Krankheitsprädisposition ließe sich mit geringem Aufwand im Rahmen einer labormäßigen Untersuchung einer Körperflüssigkeits- oder Gewebeprobe ermitteln.

Gelöst wird diese Aufgabe gemäß der vorliegenden Erfindung dadurch, daß bei einer labormäßigen Untersuchung einer Körperflüssigkeits- oder Gewebeprobe ein quantitativ analytischer Nachweis von cis-Hydroxyprolin und Derivaten hiervon erfolgt. Die vorliegende Erfindung nutzt die Erkenntnis, daß es sich bei cis-Hydroxyprolin, insbesondere bei cis-4-Hydroxyprolin um körpereigene Substanzen handelt, deren Vorkommen im Körper in Körperflüssigkeiten wie auch im Gewebe in Abhängigkeit steht zur Prädisposition für verschiedene Krankheiten. Diese Erkenntnis ist durchaus überraschend. Nicht nur, daß bisher von einer derartigen Abhängigkeit nichts bekannt war. Mehr noch, bisher war sogar unbekannt, daß es sich bei cis-Hydroxyprolin überhaupt um körpereigene Substanzen handelt. Vielmehr ging man in der Fachwelt bisher davon aus, daß im Körper eine Synthese von cis-Hydroxyprolin, insbesondere von cis-4-Hydroxyprolin nicht erfolgt. Bekannt war lediglich, daß im menschlichen Körper trans-4-Hydroxyprolin gebildet wird.

Für diese trans-Form des Hydroxyprolins ist der Wert einer labormäßigen Untersuchung einer Körperflüssigkeits- oder Gewebeprobe schon seit längerer Zeit bekannt, wobei sich der Knochenabbau, insbesondere bei Osteoporose, analytisch quantitativ bestätigen und näher erfassen läßt.

Die vorliegende Erfindung baut auf der Erkenntnis auf, daß der bisher nicht als körpereigene Substanz beobachteten cis-Form des Hydroxyprolins eine große Bedeutung bei anderen Erkrankungen zukommt, zu denen insbesondere Krebs und Kreislauferkrankungen gehören.

Die gemäß der vorliegenden Erfindung durchgeführte quantitativ analytische Bestimmung von cis-Hydroxyprolin erweist sich nicht nur als zweckmäßig, um einen Indikator für eine Krankheitsprädisposition zu ermitteln. Vielmehr kann die Erfindung auch bei bereits klinisch bestehender Erkrankung zum Einsatz kommen. Mit der quantitativen Bestimmung von cis-4-Hydroxyprolin in Körperflüssigkeiten und/oder Geweben lassen sich insbesondere erniedrigte Spiegel dieser Substanz bestimmen, die im Zusammenhang stehen können mit einer auf metabolische Ursachen zurückgehenden ungenügenden körpereigenen Synthese dieser Substanz bei Vorstadien oder Frühformen von Erkrankungen. Ebenso lassen sich in Anwendung der vorliegenden Erfindung gegenüber normalen cis-4-Hydroxyprolin-Werten erhöhte Werte nachweisen, die im Zusammenhang stehen können mit einem Gewebeumbau oder sogar Gewebezerfall.

Von besonderem Wert ist die erfindungsgemäße cis-Hydroxyprolin-Bestimmung auch im Rahmen der Verlaufskontrolle des Krankheitsgeschehens, was auch für die Beurteilung der Wirksamkeit therapeutischer Maßnahmen und ihrer Dosierung wichtig ist.

Die vorliegende Erfindung umfaßt deshalb den Nachweis und die quantitative Bestimmung des cis-Hydroxyprolins im menschlichen wie auch im Säugetierorganismus, und zwar in sämtlichen Körperflüssigkeiten wie Blut, Urin, Lymphe, Liquor cerebrospinalis, Ascites, anderen Exsudaten, aber auch in Körpergeweben.

Die Bestimmung bezieht sich nicht nur auf das cis-4-hydroxy-L-prolin, sondern auch auf die anderen Konfigurationen des cis-Hydroxyprolins wie L- und D-Form sowie eine verschiedene Ringpositionierung der Hydroxylgruppe. Sie bezieht sich auch auf Derivate des cis-Hydroxyprolins wie N-methyl-cis-Hydroxyprolin, und sie bezieht sich auf jene Peptid- oder sonstigen Verbindungen, aus denen die genannten Substanzen zum Zwecke der Untersuchung erst herausgelöst werden müssen.

Die Bestimmung der genannten Verbindung kann mit verschiedenen an sich bekannten analytischen Verfahren erfolgen, wozu die Gaschromatographie, die Säulenchromatographie, die Massenspektroskopie, das HPLC-Verfahren, die Ionenaustauschchromatographie, Immunassays, Radio-Immunassays, Enzym-Immunassays, Fluoreszenz-Immunassays und andere gehören. Bei der Spaltung von Peptid- und ähnlichen Verbindungen ist auf die Verwendung üblicher schonender Verfahren zu achten.

Ein bevorzugtes Verfahren, das im Rahmen der vorliegenden Erfindung zur Bestimmung von cis-Hydroxyprolin und dessen Derivaten in einer Körperflüssigkeits- oder Gewebeprobe einsetzen läßt, ist im Anspruch 4 angegeben, wobei bevorzugte Weiterbildungen den auf Anspruch 4 zurückbezogenen Unteransprüchen zu entnehmen sind.

Gemäß dem in Anspruch 4 angegebenen Verfahren wird zur Bestimmung von cis-Hydroxyprolin und dessen Derivaten in Körperflüssigkeiten und/oder Geweben die zu analysierende Probe zur Beseitigung störender Substanzen aufbereitet und der Gehalt an cis-Hydroxyprolin und dessen Derivaten in dieser Probe quantitativ bestimmt. Was unter dem Begriff "störende Substanz" zu verstehen ist, richtet sich nach der jeweils angewandten Bestimmungsmethode. Im Falle der Anwendung der HPLC-Methode sind als störende Substanzen solche zu verstehen, die aufgrund eines übereinstimmenden Absorptionsverhaltens bei im wesentlichen gleicher Retentionszeit die Messung des cis-Hydroxyprolins und seiner Derivate verfälschen können. Solche Substanzen sind zu eliminieren. Damit ist es möglich, cis-4-Hydroxyprolin z.B. in der im menschlichen Körper vorliegenden Konzentration von < 1 µg/ml zu bestimmen.

Besonders bevorzugt erfolgt die Bestimmung des Gehaltes an cis-Hydroxyprolin in Körperflüssigkeiten und/oder Geweben durch Vergleich mit einem externen und/oder internen Standard. Damit ist eine zuverlässige Quantifizierung der Konzentration von cis-Hydroxyprolin möglich, auch in den vorstehend genannten geringen Konzentrationen.

Für die Bestimmung des Gehalts an cis-4-Hydroxyprolin unter Anwendung der HPLC-Methode bei einer Anregungswellenlänge von 471 nm hat sich als geeigneter interner Standard cis-3-Hydroxyprolin (cis-3-HYP) erwiesen, dessen Retentionszeit (6,00 min) bei Verwendung einer geeigneten Trennsäule (z.B. RP-8) und einer günstigen Meßtemperatur (z.B. 60°C) deutlich länger ist als die von cis-4-Hydroxyprolin (4,60 min) und trans-4-Hydroxyprolin (3,30 min).

Bevorzugt erfolgt dabei die Aufbereitung der zu analysierenden Probe in mehreren Schritten, wobei die Körperflüssigkeit und/oder das Gewebe mit einem internen Standard (s.o.) versetzt, die gemäß diesem Schritt erhaltene Mischung hydrolisiert, anschließend die hydrolysierte Mischung mit mindestens einem Alkalihydroxyd und mindestens einem Alkalicarbonat versetzt, das Produkt aus diesem Schritt mit einem Störsubstanzen entfernenden Reagenz und einem Derivatisierungsreagenz behandelt und anschließend der Gehalt an cis-4-Hydroxyprolin und dessen Derivaten quantitativ bestimmt wird. Dabei hat es sich als vorteilhaft erwiesen, daß die Hydrolyse in Gegenwart einer starken Mineralsäure, insbesondere Salzsäure, bei erhöhter Temperatur, *vorzugsweise zwischen 80°C und 120°C*, besonders bevorzugt bei einer Temperatur von etwa 100°C erfolgt. Ebenso ist es besonders vorteilhaft, wenn der pH der hydrolysierten Mischung auf einen Wert zwischen 8,5 und 9,0 eingestellt wird. Als für die Erfindung geeignete basische Alkalimetallverbindungen sind die Hydroxyde, Carbonate und/oder Bicarbonate des Natriums oder Kaliums vorzugsweise einsetzbar.

Als Reagenz, mit dem Störsubstanzen entfernt werden, eignen sich insbesondere Keto-Gruppen enthaltende Verbindungen. Dabei haben sich als Keto-Verbindungen Aldehyde, insbesondere Ortho-Phthaldialdehyd mit einer Konzentration zwischen 45 und 55 g/l, insbesondere von 50 g/l als geeignet erwiesen. Nach Stehenlassen bei Raumtemperatur wird dann ggf. gebildeter Niederschlag durch Sedimentation, Zentrifugation, Filtration oder ein sonstiges geeignetes Verfahren abgetrennt.

Zu der vorstehend erhaltenen, von Niederschlag befreiten Lösung werden anschließend als Laufmittel ein wässriger Phosphatpuffer und ein Derivatisierungsreagenz zugesetzt, mit dem das zu bestimmende cis-4-Hydroxyprolin chemisch modifiziert wird. Übliche und auch im Stand der Technik bekannte Derivatisierungsreagenzien sind Azofarbstoffe, wobei Dabsylchlorid mit einer Konzentration zwischen 220 und 270 mg/l, insbesondere von 242,8 mg/l von besonderem Interesse ist. Zur Beschleunigung der Reaktion wird vorteilhafterweise das Gemisch aus zu analysierender Probe, Puffer und Derivatisierungsreagenz kurz (z.B. ca. 15 min) auf Temperaturen von ca. 70°C erwärmt.

Falls erforderlich, wird die im vorstehenden Schritt erhaltene Reaktionslösung auf Raumtemperatur abgekühlt und ggf. noch mit einer geringen Menge desselben wie im vorstehenden Schritt eingesetzten wässrigen Phosphatpuffers als Laufmittel versetzt. Die quantitative Bestimmung des cis-4-Hydroxyprolins und dessen Derivate erfolgt dann bei einer solchermaßen aufbereiteten Probe mittels HPLC.

Die Erfindung wird nachfolgend anhand der Beispiele näher erläutert:

### Beispiel 1:

Herstellung von externen/internen Standardlösungen für die Quantifizierung von cis-4-Hydroxyprolin in Körperflüssigkeiten
a) Herstellen einer externen Standardlösung: cis-4-(L)-HYP, trans-4(L)-HYP, cis-3-(DL)-HYP
   1) Stammlösung: c = 0,1 mg/ml; (gleiche Volumina einer Lösung von 5 mg cis-4-HYP sowie 5 mg trans-4-HYP in 25 ml Reinstwasser (bidestilliertes Wasser) und einer Lösung von 5 mg cis-3-HYP in 25 ml Reinstwasser werden gemischt)
   2) 1 ml HCl (6 M) und 1 ml von 1) mischen
   3) 1 ml von 2) mit 5 ml NaOH (0,5 M) und 5 ml Na₂CO₃ (0,25 M) mischen
   4) pH-Wert auf 8,5 durch Zusatz von HCl (32%, 10 M) einstellen
   5) 10 ml von 4) mit 15 ml Dabsylchlorid (c = 242,8 mg/l) mischen, 10 min auf 70°C erhitzen
   6) auf Raumtemperatur abkühlen lassen, danach mit Aceton (falls eine Trübung auftritt, werden ein paar Tropfen Reinstwasser bis zur Auflösung zugegeben) auf 25 ml auffüllen
   7) 25 ml von 6) werden mit Reinstwasser auf 146,156 ml aufgefüllt.
b) Herstellung einer internen Standardlösung (IS) :
   cis-3-(DL)-HYP
   Gleiche Volumenteile der oben verwendeten Lösung von cis-3-HYP (5 mg in 25 ml Reinstwasser) und Reinstwasser werden gemischt.

### Beispiel 2:

Quantitative Bestimmung von cis-4-HYP in Urin
1) 1 ml Probeurin wird mit 1 ml HCl (10 M) und 0,1 ml IS gemäß Beispiel 1 versetzt und 16 h bei 100°C hydrolysiert.
2) 1 ml von 1) wird mit je 0,25 ml einer wässrigen Lösung von NaOH (16 M) und Na₂CO₃ (4 M) versetzt. Ggf. wird der pH-Wert mit 10 M HCl auf pH 8,5 bis 9,0 eingestellt.
3) 0,5 ml von 2) wird mit 0,2 ml Ortho-Phthaldialdehyd (c = 50 g/l) und mit 0,2 ml Reinstwasser innig gemischt und für 60 min stehengelassen. Danach wird ggf. ausgefallener Niederschlag abzentrifugiert.
4) 0,15 ml der überstehenden Lösung von 3) werden mit 0,2 ml eines wässrigen Phosphatpuffers (22 Massen-% Acetonitril, 78 Massen-% Reinstwasser, di-Natriumhydrogenphosphat-Dodecahydrat, Citronensäure, pH = 4,7) sowie 0,2 ml Dabsylchlorid (c = 242,8 mg/l) versetzt, das Reaktionsgefäß fest verschlossen und im Trockenschrank während 15 min auf 70°C erhitzt.
5) Die Reaktionslösung von 4) wird auf Raumtemperatur abgekühlt und im Anschluß mit weiteren 0,3 ml wässrigen Phosphatpuffer als Laufmittel versetzt.
6) HPLC-Messung unter Verwendung einer RP-8-Trennsäule bei einer Meßtemperatur von 60°C im Säulenofen und einer Anregungswellenlänge von 471 nm: Injektionsvolumen: 20 µl

Untersucht wurden Urinproben von 10 männlichen und 10 weiblichen Probanden verschiedenen Alters.

In der nachfolgenden Tabelle sind die Meßergebnisse zusammen mit den Angaben über Harnstoff-, Kreatinin- und Gesamteiweiß der jeweiligen Probe aufgelistet:

**Tabelle 1:**

| Chromatographische Quantifizierung von cis-4-HYP nativen Urinen | | | | | | |
|---|---|---|---|---|---|---|
| X³ | Alter (Y), Geschlecht | Harnstoff (mg/dl) | Kreatinin (mg/dl) | U/CSF (mg/dl) | c(trans-4-HYP) (µg/ml) | c(cis-4-HYP) (µg/ml) |
| 51 | 62, w | 675 T | 34,41 | < 2 | 6,7 | 0,4 |
| 52 | 56, w | 1628 | 71,42 | 3,1 | 8,6 | 0,4 |
| 53 | 19, w | 1360 | 141,81 | 15,1 | 38,0 | 1,3 |
| 54 | 6 Tage, w | 130 T | 12,90 | 7,6 | 110 | 1,8 |
| 55 | 49, w | 410 T | 13,13 | 10,4 | 6,8 | 0,2 |
| 56 | 86, w | 474 T | 23,40 | < 2 | 4,9 | 0,3 |
| 57 | 56, w | 1726 | 105,35 | 9,4 | 24 | 1,2 |
| 58 | 83, w | 763 T | 48,03 | 5,5 | 5,8 | 0,5 |
| 59 | 33, w | 802 T | 80,23 | 2,6 | 22,8 | 0,3 |
| 60 | 65, w | 957 | 16,34 | < 2 | 12,6 | 0,2 |
| I^{b} | | | | | 8,4 | 0,6 |
| II^{b} | | | | | 9,4 | 1,4 |
| III^{b} | | | | | 8,9 | 11,6 |
| 61 | 35, m | 1866 | 151,27 | 4,6 | 26,9 | 0,5 |
| 62 | 69, m | 2480 | 51,57 | 5,3 | 22,6 | 0,9 |
| 63 | 81, m | 2197 | 91,77 | 4,8 | 45,0 | 1,5 |
| 64 | 86, m | 2068 | 130,50 | 7,8 | 24,5 | 1,7 |
| 65 | 50, m | 2221 | 194,16 | 9,1 | 31,2 | 0,9 |
| 66 | 60, m | 772 T | 120,69 | 5,4 | 20,3 | 0,8 |
| 67 | 57, m | 2597 | 171,67 | 13,4 H | 25 | 0,7 |
| 68 | 45, m | 1375 | 70,94 | < 2 | 7,6 | 0,5 |
| 69 | 35, m | 351 T | 74,06 | 12,5 H | 17 | 0,2 |
| 70 | 69, m | 337 T | 75,23 | 6,8 | 5,9 | 0,2 |
| I^{b} | | | | | 8,6 | 0,3 |
| II^{b} | | | | | 7,9 | 4,5 |
| III^{b} | | | | | 8,6 | 11,1 |

| | | | | | | |
|---|---|---|---|---|---|---|
| ^{a)} Proben-Nr-EOXO-X ^{b)} Kontrollurine I - III I: c(cis-4-HYP) = 0,3 µg/ml II: c(cis-4-HYP) = 1,4 µg/ml III: c(cis-4-HYP) = 11,2 µg/ml I - III: c(trans-4-HYP) = 8,7 µg/ml | | | | | | |

## Patentansprüche

1. Labormäßige Untersuchung einer Körperflüssigkeits- oder Gewebeprobe,
**dadurch gekennzeichnet,**
**daß** ein quantitativ analytischer Nachweis von cis-Hydroxyprolin und Derivaten hiervon erfolgt.

2. Labormäßige Untersuchung einer Körperflüssigkeits- oder Gewebeprobe gemäß Anspruch 1,
**dadurch gekennzeichnet,**
**daß** ein quantitativ analytischer Nachweis von cis-4 Hydroxyprolin erfolgt.

3. Labormäßige Untersuchung nach Anspruch 1,
**dadurch gekennzeichnet,**
**daß** der quantitativ analytische Nachweis des cis-Hydroxyprolins und seiner Derivate mittels HPLC, Säulenchromatographie, Gaschromatographie, Massenspektroskopie, Ionenaustauschchromatographie, Immunassays, Radio-Immunassays, Enzym-Immunassays, Fluoreszenz-Immunassays oder andere entsprechende Antikörper-Verfahren durchgeführt wird.

4. Verfahren zur Bestimmung von cis-Hydroxyprolin und dessen Derivaten im Rahmen einer labormäßigen Untersuchung einer Körperflüssigkeits- oder Gewebeprobe gemäß Anspruch 1,
**dadurch gekennzeichnet,**
**daß** die zu analysierenden Körperflüssigkeits- oder Gewebeprobe zur Beseitigung störender Substanzen aufbereitet und der Gehalt an cis-Hydroxyprolin und dessen Derivaten in dieser Probe quantitativ bestimmt wird.

5. Verfahren nach Anspruch 4,
**dadurch gekennzeichnet,**
**daß** die Bestimmung des cis Hydroxyprolins und dessen Derivate mittels HPLC, Gaschromatographie, Säulenchromatographie, Massenspektroskopie, Ionenaustauschchromatographie, RIA, ELISA oder Fluoreszenz-Immunassay durchgeführt wird.

6. Verfahren nach Anspruch 4,
**dadurch gekennzeichnet,**
**daß** die Bestimmung des Gehalts an cis-Hydroxyprolin und dessen Derivaten durch Vergleich mit einem externen und/oder internen Standard erfolgt.

7. Verfahren nach Anspruch 4, wobei der Gehalt der Körperflüssigkeits- oder Gewebeprobe an cis-4-Hydroxyprolin mittels HPLC bestimmt wird, umfassend die folgenden Schritte:
a) die Körperflüssigkeits- oder Gewebeprobe wird mit einem internen Standard versetzt;
b) die gemäß Schritt a) erhaltene Mischung wird hydrolysiert;
c) das gemäß Schritt b) erhaltene Produkt wird mit mindestens einem Alkalihydroxyd und mindestens einem Alkalicarbonat versetzt;
d) das gemäß Schritt c) erhaltene Produkt wird mit einem Reagenz, mit dem Störsubstanzen entfernt werden und einem Derivatisierungsreagenz versetzt; und
e) der Gehalt an cis-4-Hydroxyprolin und dessen Derivaten in dem in Schritt d) erhaltenen Produkt wird bestimmt.

8. Verfahren nach Anspruch 7,
**dadurch gekennzeichnet,**
**daß** vor Schritt b) eine Säure zugesetzt wird.

9. Verfahren nach Anspruch 8,
**dadurch gekennzeichnet,**
**daß** die Hydrolyse in Gegenwart von Salzsäure bei einer Temperatur zwischen 80°C und 120°C erfolgt.

10. Verfahren nach Anspruch 7,
**dadurch gekennzeichnet,**
**daß** die in Schritt c) eingesetzten Alkalimetallverbindungen Hydroxyde bzw. Carbonate des Natriums oder Kaliums sind.

11. Verfahren nach Anspruch 7,
**dadurch gekennzeichnet,**
**daß** der pH-Wert in Schritt c) durch Zugabe von HCl auf pH 8,5 bis 9 eingestellt wird.

12. Verfahren nach Anspruch 7,
**dadurch gekennzeichnet,**
**daß** in Schritt d) Ortho-Phthaldialdehyd (OPA) und als Derivatisierungsreagenz ein Azofarbstoff eingesetzt wird.

13. Verfahren nach Anspruch 7,
**dadurch gekennzeichnet,**
**daß** von der quantitativen Bestimmung des cis-4-Hydroxyprolins und dessen Derivate in Schritt e) die Temperatur erniedrigt wird.

14. Verfahren nach Anspruch 4,
**dadurch gekennzeichnet,**
**daß** die Körperflüssigkeitsprobe in einer Urinprobe oder Blutprobe besteht.

15. Verfahren nach Anspruch 7,
**dadurch gekennzeichnet,**
**daß** als Interner Standard (IS) cis-3-Hydroxyprolin eingesetzt wird.

16. Analysekit zur Durchführung des Verfahrens gemäß Anspruch 7, umfassend HCl (10 M), NaOH (16 M), Na₂CO₃ (4 M), Ortho-Phthaldialdehyd, wässrigen Phosphat-Puffer und Dabsylchlorid.

17. Analysekit nach Anspruch 16,
**dadurch gekennzeichnet,**
**daß** die Konzentration des Ortho-Phthalaldehyds zwischen 45 und 55 g/l beträgt.

18. Analysekit nach Anspruch 16,
**dadurch gekennzeichnet,**
**daß** die Konzentration des Dabsylchlorids zwischen 220 und 270 mg/l beträgt.

19. Analysekit nach Anspruch 16,
**dadurch gekennzeichnet,**
**daß** er mindestens eine RP-8-Trennsäule umfaßt.

## Claims

1. Laboratory test of a body fluid or tissue sample,
**characterized in**
**that** cis-hydroxyproline and derivatives thereof are detected by means of quantitative analysis.

2. Laboratory test of a body fluid or tissue sample, as claimed in claim 1,
**characterized in**
**that** cis-4-hydroxyproline is detected by means of quantitative analysis.

3. Laboratory test of a body fluid or tissue sample, as claimed in claim 1,
**characterized in**
**that** the quantitatively analytic detection of cis-hydroxyproline and its derivatives is carried out by means of HPLC, column chromatography, gas chromatography, mass spectroscopy, ion exchange chromatography, immunoassay, radio immunoassay, enzyme immunoassay, fluorescence immunoassay or other corresponding antibody methods.

4. Process for determining cis-hydroxyproline and its derivatives for the purpose of a laboratory test of a body fluid or tissue sample, as claimed in claim 1,
**characterized in**
**that** the body fluid or tissue sample to be analyzed is prepared to eliminate disturbing substances; and that the cis-hydroxyproline and its derivative content is determined quantitatively in this sample.

5. Process, as claimed in claim 4,
**characterized in**
**that** the determination of cis-hydroxyproline and its derivatives is performed by means of HPLC, gas chromatography, column chromatography, mass spectroscopy, ion exchange chromatography, RIA, ELISA or fluorescence immunoassay.

6. Process, as claimed in claim 4,
**characterized in**
**that** the cis-hydroxyproline and its derivative content is determined by means of comparison with an external and / or internal standard.

7. Process, as claimed in claim 4, wherein the cis-4-hydroxyproline content in the body fluid and tissue sample is determined by means of HPLC, comprising the following steps:
a) An internal standard is added to the body fluid and / or the tissue sample.
b) The mixture, obtained according to step a), is hydrolized.
c) At least one alkali hydroxide and at least one alkali carbonate are added to the product, obtained according to step b).
d) A reagent eliminating the disturbing substance and a derivatization reagent are added to the product, obtained from step c); and
e) the cis-4-hydroxyproline and its derivative content is determined in the product obtained in step d).

8. Process, as claimed in claim 7,
**characterized in**
**that** before step b) an acid is added.

9. Process, as claimed in claim 8,
**characterized in**
**that** hydrolysis takes place in the presence of hydrochloric acid at a temperature ranging from 80 degrees C to 120 degrees C.

10. Process, as claimed in claim 7,
**characterized in**
**that** the alkali metal compounds added in step c) are hydroxides or carbonates of sodium or potassium.

11. Process, as claimed in claim 7,
**characterized in**
**that** the pH value in step c) is adjusted to a pH ranging from 8.5 to 9 with the addition of HCl.

12. Process, as claimed in claim 7,
**characterized in**
**that** in step d) ortho-phthaldialdehyde (OPA) and as the derivatization reagent an azo dye are added.

13. Process, as claimed in claim 7,
**characterized in**
**that** prior to the quantitative analysis of cis-4-hydroxyproline and its derivatives in step e) the temperature is lowered.

14. Process, as claimed in claim 4,
**characterized in**
**that** the body fluid sample is a urine sample or a blood sample.

15. Process, as claimed in claim 7,
**characterized in**
**that** cis-3-hydroxyproline is used as the internal standard (IS).

16. Analysis kit to carry out the process, as claimed in claim 7, comprising HCl (10 M), NaOH (16 M), Na₂CO₃ (4 M), ortho-phthaldialdehyde, aqueous phosphate buffer and dabsyl chloride.

17. Analysis kit, as claimed in claim 16,
**characterized in**
**that** the concentration of ortho-phthalaldehyde ranges from 45 to 55 g/l.

18. Analysis kit, as claimed in claim 16,
**characterized in**
**that** the concentration of dabsyl chloride ranges from 220 to 270 mg/l.

19. Analysis kit, as claimed in claim 16,
**characterized in**
**that** it includes at least one RP 8 separating column.

## Revendications

1. Examen en laboratoire d'un échantillon de tissu ou de liquide corporel, **caractérisé en ce que** l'on effectue une détection analytique quantitative de cis-hydroxyproline et de ses dérivés.

2. Examen en laboratoire d'un échantillon de tissu ou de liquide corporel, conforme à la revendication 1, **caractérisé en ce que** l'on effectue une détection analytique quantitative de cis-4-hydroxyproline.

3. Examen de laboratoire conforme à la revendication 1, **caractérisé en ce que** la détection analytique quantitative de la cis-hydroxyproline et de ses dérivés est effectuée par HPLC, chromatographie sur colonne, chromatographie en phase gazeuse, spectrométrie de masse, chromatographie par échange d'ions, dosage immunologique, dosage radioimmunologique, dosage immunoenzymatique, dosage par immunofluorescence ou d'autres procédés correspondants avec anticorps.

4. Procédé de dosage de cis-hydroxyproline et de ses dérivés dans le cadre d'un examen en laboratoire d'un échantillon de tissu ou de liquide corporel, conforme à la revendication 1, **caractérisé en ce que** l'on prépare l'échantillon de tissu ou de liquide corporel à analyser pour en éliminer les substances gênantes et l'on détermine quantitativement la teneur de cet échantillon en cis-hydroxyproline et en dérivés de celle-ci.

5. Procédé conforme à la revendication 4, **caractérisé en ce que** le dosage de la cis-hydroxyproline et de ses dérivés est effectué par HPLC, chromatographie sur colonne, chromatographie en phase gazeuse, spectrométrie de masse, chromatographie par échange d'ions, dosage radioimmunologique, dosage immunoenzymatique ELISA ou dosage par immunofluorescence.

6. Procédé conforme à la revendication 4, **caractérisé en ce que** le dosage de la cis-hydroxyproline et de ses dérivés est effectué par comparaison avec une référence interne et/ou externe.

7. Procédé conforme à la revendication 4, dans lequel la teneur en cis-hydroxyproline de l'échantillon de tissu ou de liquide corporel est déterminée par HPLC, et qui comporte les étapes suivantes :
a) ajouter une référence interne à l'échantillon de tissu ou de liquide corporel ;
b) hydrolyser le mélange obtenu dans l'étape (a) ;
c) ajouter au produit obtenu dans l'étape (b) au moins un hydroxyde de métal alcalin et au moins un carbonate de métal alcalin ;
d) ajouter au produit obtenu dans l'étape (c) un réactif qui permet d'éliminer les substances gênantes et un réactif de dérivatisation ;
e) et déterminer la teneur en cis-hydroxyproline et ses dérivés du produit obtenu dans l'étape (d).

8. Procédé conforme à la revendication 7, **caractérisé en ce que**, avant l'étape (b), on ajoute un acide.

9. Procédé conforme à la revendication 8, **caractérisé en ce que** l'on effectue l'hydrolyse en présence d'acide chlorhydrique et à une température de 80 à 120 °C.

10. Procédé conforme à la revendication 7, **caractérisé en ce que** les composés de métal alcalin ajoutés lors de l'étape (c) sont les hydroxydes et carbonates de sodium ou de potassium.

11. Procédé conforme à la revendication 7, **caractérisé en ce que**, lors de l'étape (c), on ajuste le pH à une valeur de 8,5 à 9, par addition d'HCl.

12. Procédé conforme à la revendication 7, **caractérisé en ce que**, lors de l'étape (d), on utilise du dialdéhyde ortho-phtalique (DAOP), ainsi qu'un colorant azoïque comme réactif de dérivatisation.

13. Procédé conforme à la revendication 7, **caractérisé en ce que**, à partir de la détermination quantitative de la cis-4-hydroxyproline et de ses dérivés effectuée lors de l'étape (e), on abaisse la température.

14. Procédé conforme à la revendication 4, **caractérisé en ce que** l'échantillon de liquide corporel est un échantillon d'urine ou de sang.

15. Procédé conforme à la revendication 7, **caractérisé en ce que** l'on emploie de la cis-3-hydroxyproline en tant que référence interne.

16. Trousse d'analyse permettant de mettre en oeuvre un procédé conforme à la revendication 7, qui comprend du HCl (10 M), de la NaOH (16 M), du Na₂CO₃ (4 M), du dialdéhyde ortho-phtalique, du tampon phosphate aqueux et du chlorure de dabsyle.

17. Trousse d'analyse conforme à la revendication 16, **caractérisée en ce que** la concentration du dialdéhyde ortho-phtalique vaut de 45 à 55 g/l.

18. Trousse d'analyse conforme à la revendication 16, **caractérisée en ce que** la concentration du chlorure de dabsyle vaut de 220 à 270 mg/l.

19. Trousse d'analyse conforme à la revendication 16, **caractérisée en ce qu'**elle comprend au moins une colonne de séparation RP-8.
